# EUROPEAN PATENT SPECIFICATION

(11) **EP 3 883 487 B1**
(45) Date of publication and mention of the grant of the patent: **06.11.2024**
(21) Application number: 19805754.9
(22) Date of filing: 11.11.2019
(51) Int. Cl.: A61B 18/14, A61B 5/06, A61B 34/20, A61B 5/287, A61B 5/00, A61B 18/00, A61B 17/00, A61B 90/00

(54) **ABLATION CATHETER WITH STACKED CIRCUIT ASSEMBLY**
ABLATIONSKATHETER MIT GESTAPELTER SCHALTUNGSANORDNUNG
CATHÉTER D'ABLATION AVEC ENSEMBLE CIRCUIT EMPILÉ

(30) Priority: 21.11.2018 US 201862770527 P
(43) Date of publication of application: 29.09.2021
(73) Proprietor: Biosense Webster (Israel) Ltd., Yokneam 2066717 (IL)
(72) Inventor: ROUSU, Corey M., Irwindale, California 91706 (US)
(74) Representative: Carpmaels & Ransford LLP
(86) International application number: PCT/IB2019/059667
(87) International publication number: WO 2020/104888

(56) References cited:
- EP-A1- 3 372 149
- US-A1- 2006 100 618
- US-A1- 2014 364 848
- US-A1- 2018 110 562
- US-A1- 2018 183 159

## Description

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of energy (e.g., radiofrequency (RF) energy), it may be possible to cease or modify the propagation of unwanted electrical signals from one portion of the heart to another. The ablation process may provide a barrier to unwanted electrical pathways by creating electrically insulative lesions or scar tissue.

In some procedures, a catheter with one or more RF electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with RF energy to thereby ablate the contacted tissue. In some cases, the electrodes may be bipolar. In some other cases, a monopolar electrode may be used in conjunction with a ground pad that is in contact with the patient.

Examples of ablation catheters are described in U.S. Pub. No. 2013/0030426, entitled "Integrated Ablation System using Catheter with Multiple Irrigation Lumens," published January 31, 2013; U.S. Pub. No. 2017/0312022, entitled "Irrigated Balloon Catheter with Flexible Circuit Electrode Assembly," published November 2, 2017; U.S. Pub. No. 2018/0071017, entitled "Ablation Catheter with a Flexible Printed Circuit Board," published March 15, 2018; U.S. Pub. No. 2018/0056038, entitled "Catheter with Bipole Electrode Spacer and Related Methods," published March 1, 2018; U.S. Pat. No. 10,130,422, entitled "Catheter with Soft Distal Tip for Mapping and Ablating Tubular Region," issued November 20, 2018; U.S. Pat. No. 8,956,353, entitled "Electrode Irrigation Using Micro-Jets," issued February 17, 2015; and U.S. Pat. No. 9,801,585, entitled "Electrocardiogram Noise Reduction," issued October 31, 2017. As described in several of the foregoing references, irrigation may be used to draw heat from ablating components of an ablation catheter; and to prevent the formation of blood clots near the ablation site.

Some catheter ablation procedures may be performed using electrophysiology (EP) mapping. Such EP mapping may include the use of sensing electrodes on a catheter (e.g., the same catheter that is used to perform the ablation). Such sensing electrodes may monitor electrical signals within the cardiovascular system to pinpoint the location of aberrant conductive tissue sites that are responsible for the arrhythmia. Examples of an EP mapping system are described in U.S. Pat. No. 5,738,096, entitled "Cardiac Electromechanics," issued April 14, 1998. Examples of EP mapping catheters are described in U.S. Pat. No. 9,907,480, entitled "Catheter Spine Assembly with Closely-Spaced Bipole Microelectrodes," issued March 6, 2018; U.S. Pat. No. 10,130,422, entitled "Catheter with Soft Distal Tip for Mapping and Ablating Tubular Region," issued November 20, 2018; and U.S. Pub. No. 2018/0056038, entitled "Catheter with Bipole Electrode Spacer and Related Methods," published March 1, 2018.

When using an ablation catheter, it may be desirable to ensure that the one or more electrodes of the ablation catheter are sufficiently contacting target tissue. For instance, it may be desirable to ensure that the one or more electrodes are contacting target tissue with enough force to effectively apply RF ablation energy to the tissue; while not applying a degree of force that might tend to undesirably damage the tissue. To that end, it may be desirable to include one or more force sensors or pressure sensors to detect sufficient contact between one or more electrodes of an ablation catheter and target tissue. Examples of ablation catheters that include integral force sensors are described in U.S. Pat. No. 8,529,476, entitled "Catheter with Strain Gauge Sensor," issued September 10, 2013, and U.S. Pub. No. 2018/0071017, entitled "Ablation Catheter with a Flexible Printed Circuit Board," published March 15, 2018.

In addition to using force sensing or EP mapping, some catheter ablation procedures may be performed using an image guided surgery (IGS) system. The IGS system may enable the physician to visually track the location of the catheter within the patient, in relation to images of anatomical structures within the patient, in real time. Some systems may provide a combination of EP mapping and IGS functionalities, including the CARTO 3^{®} system by Biosense Webster, Inc. of Irvine, California. Examples of catheters that are configured for use with an IGS system are disclosed in U.S. Pat. No. 5,391,199, entitled "Apparatus and Method for Treating Cardiac Arrhythmias," issued February 21, 1995; U.S. Pat. No. 6,690,963, entitled "System for Determining the Location and Orientation of an Invasive Medical Instrument," issued February 10, 2004; and U.S. Pat. No. 9,480,416, entitled "Signal Transmission Using Catheter Braid Wires," issued November 1, 2016; and various other references that are cited herein.

Some ablation catheters may include a distal end formed of one or more flexible printed circuit boards. Examples of such ablation catheters are described in U.S. Pub. No. 2018/0071017, entitled "Ablation Catheter with a Flexible Printed Circuit Board," published March 15, 2018; U.S. Pub. No. 2018/0110562, entitled "Catheter Distal End Made of Plastic Tube and Flexible Printed Circuit Boards," published April 26, 2018; and U.S. Pat. App. No. 15/786,968, entitled "High-Volume Manufacturing of Catheters Comprising Electrodes Having Low Impedance at Low Frequency," filed October 18, 2017.

Other devices that are part of the background prior art are shown and described in U.S Pat. No. 6,660,584, entitled "Selective Polysilicon Stud Growth of 6F2 Memory Cell Manufacturing Having a Convex Upper Surface Profile," issued December 9, 2003; U.S Pat. No. 7,047,059, entitled "Simplified Water-Bag Technique for Magnetic Susceptibility Measurements on the Human Body and Other Specimens," issued May 16, 2006; U.S Pat. No. 7,212,864, entitled "Modular Implantable Medical Device," issued May 1, 2007; U.S Pat. No. 9,061,134, entitled "Systems and Methods for Flexible Electrodes," issued June 23, 2015; and U.S. Pub. No. 2016/0151621, entitled "Implantable Medical Device with Stacked Circuit Components," issued June 2, 2016.

While several catheter systems and methods have been made and used, it is believed that no one prior to the inventors has made or used the invention described, illustrated and claimed herein.

US 2006/100618 A1 discloses an ablation catheter having distal and proximal ends for performing ablation on a human tissue region comprising at least one electrode.

US 2018/183159 A1 discloses a multi-line electrical assembly that includes a multi-line cable, including a multiplicity of insulated conductive wires; and a multi-line electrical plug, physically connected to the cable.

US 2014/364848 A1 discloses a system for diagnosis or treatment of tissue in a body.

### SUMMARY OF THE INVENTION

The present invention provides an apparatus according to claim 1 and a manufacturing method according to claim 16. Further embodiments are defined in the dependent claims.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of a distal portion of the catheter of FIG. 1, with additional components shown in schematic form;
FIG. 3 depicts a perspective view of the distal portion of the catheter of FIG. 1, with an outer sheath omitted to reveal internal components;
FIG. 4 depicts an exploded perspective view of the distal portion of the catheter of FIG. 1;
FIG. 5 depicts an exploded perspective view of a distal tip assembly of the distal portion of the catheter of FIG. 1;
FIG. 6 depicts a perspective view of an exemplary alternative distal tip member that may be incorporated into the distal portion of the catheter of FIG. 1, with the distal tip member in a flat configuration;
FIG. 7 depicts a perspective view of a strain gauge assembly of the distal portion of the catheter of FIG. 1;
FIG. 8 depicts an exploded perspective view of the strain gauge assembly of FIG. 7;
FIG. 9 depicts a perspective view of a navigation sensor assembly of the distal portion of the catheter of FIG. 1, with the navigation sensor assembly in a flat configuration;
FIG. 10 depicts a perspective view of the navigation sensor assembly of FIG. 9 in a bent configuration;
FIG. 11 depicts a perspective view of a distal spacer of the of the distal portion of the catheter of FIG. 1;
FIG. 12 depicts a top plan view of the distal spacer of FIG. 11;
FIG. 13 depicts a perspective view of a stack of the distal spacers of FIG. 11 in combination with a pair of proximal spacers and other components of the distal portion of the catheter of FIG. 1;
FIG. 14 depicts another perspective view of a stack of the distal spacers of FIG. 11 in combination with the pair of proximal spacers and other components of FIG. 13;
FIG. 15 depicts a perspective view of a proximal spacer of the of the distal portion of the catheter of FIG. 1;
FIG. 16 depicts a top plan view of the proximal spacer of FIG. 15;
FIG. 17 depicts a perspective view of an exemplary alternative navigation sensor assembly that may be incorporated into the distal portion of the catheter of FIG. 1, with the navigation sensor assembly in a flat configuration; and
FIG. 18 depicts a perspective view of the navigation sensor assembly of FIG. 17 in a bent configuration.

### DETAILED DESCRIPTION FOR MODES OF CARRYING OUT THE INVENTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g. "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

### I. Exemplary Ablation Catheter System

### A. Overview

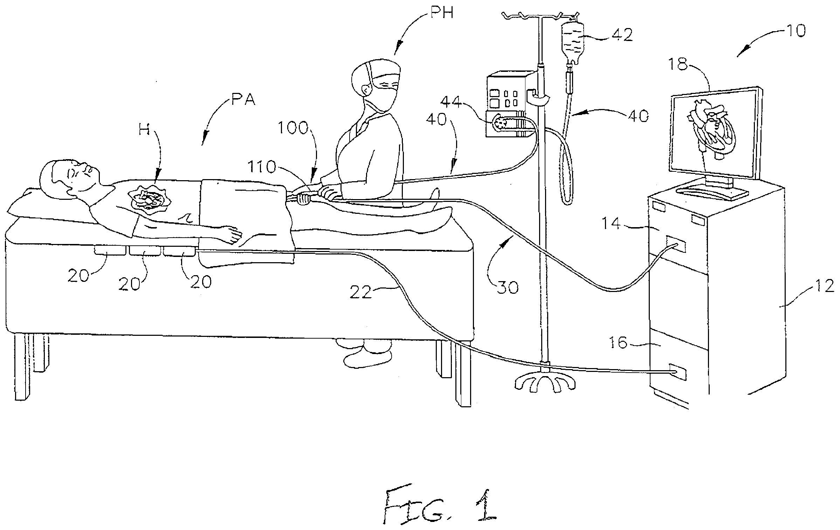

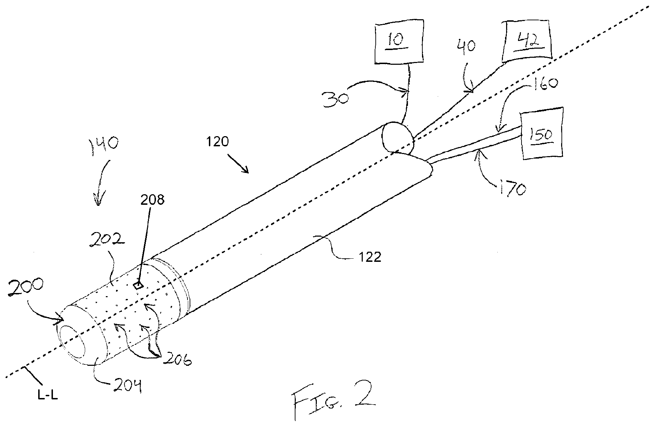

FIG. 1 shows an exemplary medical procedure and associated components of a cardiac ablation catheter system that may be used to provide cardiac ablation as referred to above. In particular, FIG. 1 shows a physician (PH) grasping a handle (110) of a catheter assembly (100), with an end effector (140) of a catheter (120) (shown in FIG. 2 but not shown in FIG. 1) of catheter assembly (100) disposed in a patient (PA) to ablate tissue in or near the heart (H) of the patient (PA). As shown in FIG. 2, catheter (120) includes an elongate flexible sheath (122), with end effector (140) being disposed at a distal end of sheath (122). End effector (140) and various components that are contained in sheath (122) will be described in greater detail below. Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). As described below, field generators (20) may also be referred to as external transmitter coils (TX). Field generators (20) are merely optional.

Guidance and drive system (10) of the present example includes a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via microelectrodes (208) of end effector (140) as described in greater detail below. Console (12) includes a processor (not shown) that processes such EP mapping signals and thereby provides EP mapping as is known in the art.

First driver module (14) of the present example is further operable to provide RF power to a distal tip member (200) of end effector (140), as will be described in greater detail below, to thereby ablate tissue.

Second driver module (16) is coupled with field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

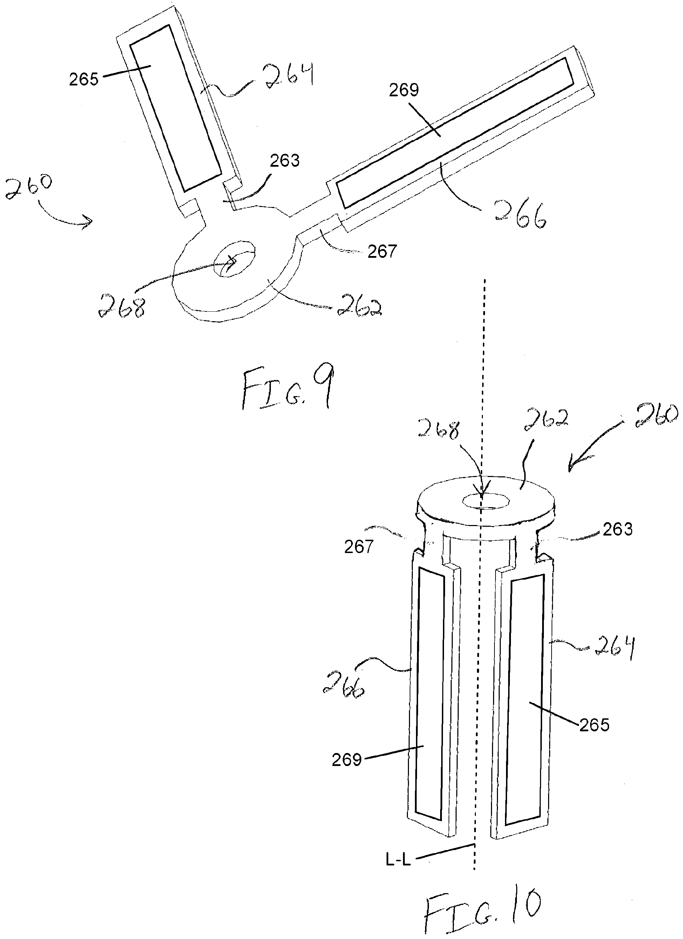

First driver module (14) is also operable to receive position indicative signals from a navigation sensor assembly (260) in end effector (140). In such versions, the processor of console (12) is also operable to process the position indicative signals from navigation sensor assembly (260) to thereby determine the position of end effector (140) within the patient (PA). As will be described in greater detail below with reference to FIGS. 9-10, navigation sensor assembly (260) includes a pair of coils (265, 269) (shown schematically in FIGS. 9-10) that are operable to generate signals that are indicative of the position and orientation of end effector (140) within the patient (PA). Coils (265, 269) are configured to generate electrical signals in response to the presence of an alternating electromagnetic field generated by field generators (20). Other components and techniques that may be used to generate real-time position data associated with end effector (140) may include wireless triangulation, acoustic tracking, optical tracking, inertial tracking, and the like. By way of example only, position sensing may be provided in accordance with at least some of the teachings of U.S. Pat. No. 5,391,199; U.S. Pat. No. 6,690,963; or U.S. Pat. No. 9,480,416. Alternatively, end effector (140) may lack a navigation sensor assembly (260).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from navigation sensor assembly (260) of end effector (140). For instance, as end effector (140) of catheter (120) moves within the patient (PA), the corresponding position data from navigation sensor assembly (260) may cause the processor of console (12) to update the patient anatomy views in display (18) in real time to depict the regions of patient anatomy around end effector (140) as end effector (130) moves within the patient (PA). Moreover, the processor of console (12) may drive display (18) to show locations of aberrant conductive tissue sites, as detected via electrophysiological (EP) mapping with end effector (140) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of end effector (140) on the images of the patient's anatomy, such as by superimposing an illuminated dot, a crosshair, a graphical representation of end effector (140), or some other form of visual indication. Such a superimposed visual indication may also move within the images of the patient anatomy on display (18) in real time as the physician moves end effector (140) within the patient (PA), thereby providing real-time visual feedback to the operator about the position of end effector (140) within the patient (PA) as end effector (140) moves within the patient (PA). The images provided through display (18) may thus effectively provide a video tracking the position of end effector (140) within a patient (PA), without necessarily having any optical instrumentation (i.e., cameras) viewing end effector (140). In the same view, display (18) may simultaneously visually indicate the locations of aberrant conductive tissue sites detected through EP mapping. The physician (PH) may thus view display (18) to observe the real time positioning of end effector (140) in relation to the mapped aberrant conductive tissue sites and in relation to images of the adjacent anatomical structures in the patient (PA).

Fluid source (42) of the present example includes a bag containing saline or some other suitable irrigation fluid. Conduit (40) includes a flexible tube that is further coupled with a pump (44), which is operable to selectively drive fluid from fluid source (42) to catheter assembly (100). As described in greater detail below, such irrigation fluid may be expelled through openings (206) of distal tip member (200) of end effector (140). Such irrigation may be provided in accordance with the teachings of any of the various patent references cited herein; or in any other suitable fashion as will be apparent to those skilled in the art in view of the teachings herein.

### B. Exemplary Stacked Circuit Assembly for Distal Portion of Ablation Catheter

In some instances, it may be desirable to form assemblies using a stack of discretely formed circuits, such as by stacking discretely formed circuit boards. The circuits within the stack may be coupled together (e.g., by vias, etc.) to thereby collectively form one or more circuits in the stack. By forming an assembly out of stacked circuits, the mechanical and electrical properties of each layer in the stack may be precisely controlled using chemical etching to form the circuits, pads, vias, or other features; then laser cutting each layer to form the desired shape for each layer. In instances where a layer of the stack requires a three-dimensional shape, the layer may be initially constructed in a flat, two-dimensional profile; then formed into a three-dimensional configuration using secondary processes (e.g., bending, etc.).

With each layer in the stack having its own circuit components, the layers can be electrically coupled together to provide electrical continuity throughout the entire stack through the use of vias or other conductive features. By way of example only, vias connecting traces between the layers may be laser drilled, mechanically drilled, or otherwise formed. By way of further example only, such vias may have inner diameters ranging from approximately 0.001 inches to approximately 0.025 inches.

While electrical circuit components in some layers may be electrically coupled with circuit components of electrical circuit components of other layers (e.g., through vias), some other electrical circuit components may remain electrically isolated from others. For instance, circuit components associated with providing RF ablation through distal tip member (200) may be electrically isolated from circuit components associated with providing EP mapping through microelectrodes (208). Similarly, circuit components associated with providing RF ablation and EP mapping may be electrically isolated from circuit components associated with providing position-indicative signals from navigation sensor assembly (260).

By using stacked layers of circuits to form end effector (140) or other components of the distal portion of catheter (120), as opposed to using conventional catheter manufacturing techniques, the cost to manufacture catheter (120) may be reduced. In addition, or in the alternative, using stacked layers of circuits to form end effector (140) or other components of the distal portion of catheter (120), as opposed to using conventional catheter manufacturing techniques, may speed up the process of manufacturing catheter (120). The following describes how stacked layers of circuits may be used to form end effector (140) or other components of the distal portion of catheter (120).

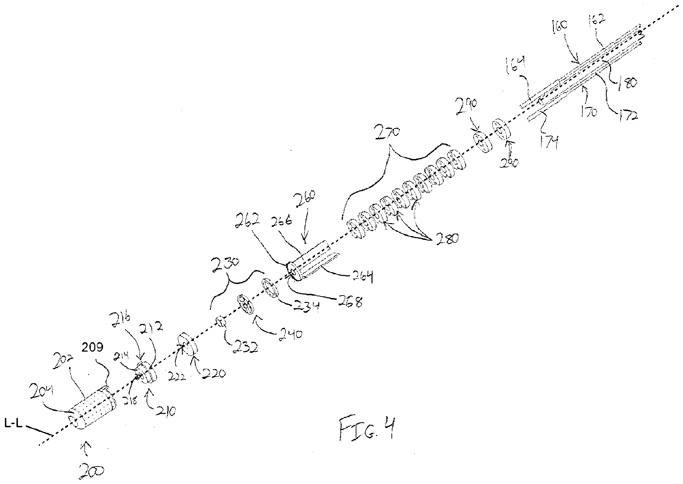

FIGS. 2-4 show the components of end effector (140), and other components of the distal portion of catheter (120), in greater detail. As shown, end effector (140) is formed by a stack of various layers. Each of these layers provides a piece of the circuit formed by the stack. These layers may be etched and cut from a single sheet, then stacked and bonded together using conventional bonding techniques. In some instances, each layer may include one or more integral tabs that are used to grasp and position the layers in the stack. After the layers are bonded together in the stack such tabs may be removed using conventional techniques. Those skilled in the art will also recognize that the layers may include holes or other features that are used to facilitate proper alignment of the layers with each other.

As shown in FIGS. 2-4, end effector (140) includes a distal tip member (200), a distal tip base (210), a distal circuit disk (220), a strain gauge assembly (230), a navigation sensor assembly (260), a distal spacer stack (270), and a pair of proximal spacers (290). Distal tip member (200), distal tip base (210), distal circuit disk (220), strain gauge assembly (230), navigation sensor assembly (260), distal spacer stack (270), and proximal spacers (290) are coaxially aligned with each other and are stacked longitudinally so that these components (210-290) define a stacked circuit. A pair of push-pull cables (160, 170) and an irrigation tube (180) extend along the length of catheter (120) to reach end effector (140). Each of the foregoing components will be described in greater detail below. Flexible sheath (122) surrounds all of the foregoing components except for distal tip member (200).

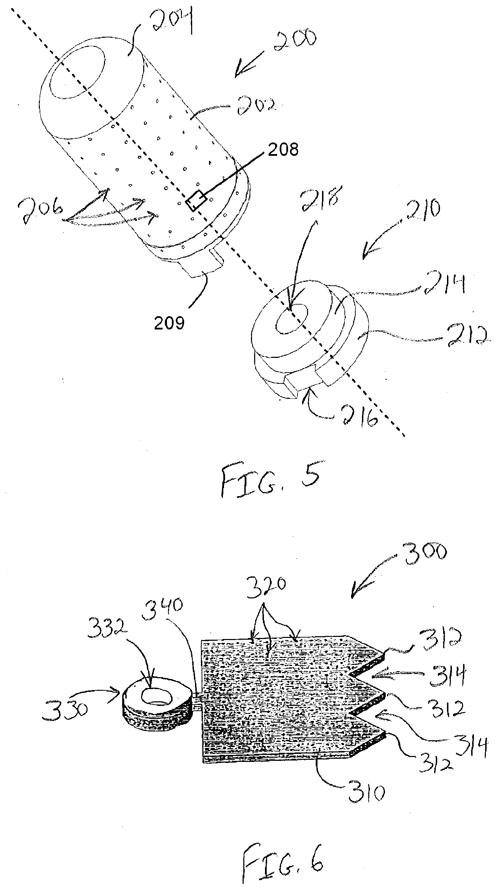

FIG. 5 shows distal tip member (200) and distal tip base (210) in greater detail. As shown, distal tip member (200) of the present example includes a cylindraceous body (202) with a dome tip (204). Cylindraceous body (202) and dome tip (204) may be formed of an electrically conductive material, such as metal. A plurality of openings (206) are formed through cylindraceous body (202) and are in communication with the hollow interior of distal tip member (200). Openings (206) thus allow irrigation fluid to be communicated from the interior of distal tip member (200) out through cylindraceous body (202), thereby enabling distal tip member (200) to expel irrigation fluid within a patient (e.g., within a cardiovascular structure). Cylindraceous body (202) and dome tip (204) are also operable to apply RF electrical energy to tissue to thereby ablate the tissue. Such RF electrical energy may be communicated from first driver module (14) to the proximal-most spacer (290) via cable (30). Structures that may be used to communicate such electrical energy from the proximal-most spacer (290) to distal tip member (200) are described elsewhere herein. Distal tip member (200) may also receive such RF electrical energy in accordance with the teachings of various patent references cited herein.

Distal tip member (200) of the present example also includes one or more EP mapping microelectrodes (208) mounted to cylindraceous body (202). EP mapping microelectrodes (208) are configured to pick up electrical potentials from tissue that comes into contact with EP mapping microelectrodes (208). EP mapping microelectrodes (208) may thus be used to determine locations of aberrant electrical activity in tissue within a cardiovascular anatomical structure (e.g., pulmonary vein, etc.). Signals picked up by EP mapping microelectrodes (208) may be communicated through vias or other structures in the layers that are proximal to strain gauge assembly (230), eventually reaching first driver module (14) of console (12) via cable (30). First driver module (14) may process the EP mapping signals and provide the physician (PH) with corresponding feedback indicating the locations of aberrant electrical activity in accordance with the teachings of various references cited herein.

In versions where cylindraceous body (202) is formed of an electrically conductive material to provide RF electrical energy for tissue ablation, an electrically insulating material may be interposed between cylindraceous body (202) and EP mapping microelectrodes (208) to thereby electrically isolate EP mapping microelectrodes (208) from cylindraceous body (202). EP mapping microelectrodes (208) may be constructed and operable in accordance with the teachings of various patent references cited herein.

While only one EP mapping microelectrode (208) is shown, distal tip member (200) may include two or more EP mapping microelectrodes (208). Alternatively, distal tip member (200) may lack EP mapping microelectrodes (208) altogether. In some other variations, end effector (140) includes one or more ring electrodes, proximal to distal tip member (200), to provide EP mapping functionality. Some variations of end effector (140) may lack EP mapping functionality.

Distal tip base (210) includes a proximal body portion (212) and a distal body portion (214), with a central aperture (218) formed through both body portions (212, 214). Central aperture (218) is configured to provide a path for communication of irrigation fluid to the hollow interior of distal tip member (200), as will be described in greater detail below. By way of example only, aperture (218) may have an inner diameter ranging from approximately 0.030 inches to approximately 0.050 inches. The outer diameter of proximal body portion (212) is greater than the outer diameter of distal body portion (214), such that distal tip base (214) forms an annular shoulder that the proximal edge of distal tip member (200) may abut. Proximal body portion (212) also defines a lateral notch (216) that is configured to receive a proximally extending tab (209) of distal tip member (200).

As shown in FIGS. 2-3, a distal circuit disk (220) is positioned proximal to distal tip base (210). Distal circuit disk (220) includes circuitry that is operable to communicate RF electrical energy to distal tip member (200). In versions where one or more EP mapping electrodes (208) are included, distal circuit disk (220) may also include circuitry that is operable to communicate EP mapping signals from EP mapping electrodes (208). Distal circuit disk (220) includes features that electrically interface with complementary features of proximally extending tab (209) of distal tip member (200), such that the electrical communication features of tab (209) provide a path for electrical communication between distal tip member (200) and distal circuit disk (220). By way of example only, distal circuit disk (220) may be constructed of multiple layers including polyether ether ketone (PEEK), polyimide, or liquid crystal polymer. By way of further example only, each layer of distal circuit disk (220) may have a thickness ranging from approximately 0.001 inches to approximately 0.004 inches, thereby providing distal circuit disk (220) with an overall thickness ranging from approximately 0.004 inches to approximately 0.016 inches.

In some versions, distal circuit disk (220) further includes one or more transmission coils. Such transmission coils may provide wireless communication of signals (e.g., EP mapping signals from microelectrodes (208)) to one or more complementary coils that are proximal to distal circuit disk (220). In addition, or in the alternative, such transmission coils may provide wireless communication of RF electrical energy from one or more complementary coils that are proximal to distal circuit disk (220) to distal tip member (200, 300). In versions where coils are incorporated into distal circuit disk (220) and one or more other layers that are proximal to strain gauge assembly (230), such coils may thus enable wireless communication of electrical signals across strain gauge assembly (230) without requiring wires, vias, or other electrically conductive structures to pass longitudinally across strain gauge assembly (230).

In some versions, distal circuit disk (220) includes at least one transmission coil (TX) that is paired with receiving coil (RX) of central annular body (262) to detect strain being applied to respective strain gauges (248) so as to determine the contact force applied to distal tip (200). By way of example only, some versions may provide three TX coils on distal circuit disk (220) for pairing with respective strain gauges (248) and respective RX coils formed on central annular body (262). Some other versions of distal circuit disk (220) may simply omit a TX coil.

While distal tip member (200) and distal circuit disk (220) are shown as being separate pieces in this example, in some other versions distal tip member (200) and distal circuit disk (220) are unitarily formed together as a single piece, with tab (209) extending unitarily therebetween. In such versions, the combination of distal tip member (200) and distal circuit disk (220) may simply be wrapped around distal tip base (210), with tab (209) still fitting in notch (216) of distal circuit disk (220).

FIG. 6 shows an exemplary alternative form that distal tip member (200) may take. In particular, FIG. 6 shows a flat distal tip member (300) that may be formed as a flex-circuit, with a flat and flexible body portion (310) having a plurality of openings (320) formed therethrough. One side of body portion (310) includes a set of peaks (312) and valleys (314) that form a sawtooth configuration. The opposite side of body portion (310) includes a stem (340) integrally joining body portion (310) with a disk (330). Disk (330) defines a central opening (332). Distal tip member (300) of this example may be formed using any suitable conventional flex-circuit forming techniques. From the flat configuration shown in FIG. 6, distal tip member (300) may be bent to roll body portion (310) into a cylindraceous shape, with peaks (312) being deflected inwardly toward each other to form a dome shape. By way of example only, an external metal scaffold (or other kind of scaffold) may be used to assist in appropriately bending body portion (310) into a cylindraceous shape with a distal dome feature. As another merely illustrative example, an internal mandrel may be used to assist in appropriately bending body portion (310) into a cylindraceous shape with a distal dome feature. In some such versions, the mandrel may be dissolved or otherwise disposed of after body portion (310) has achieved the desired structural configuration. Also in some versions, a cap member may be secured over the formed body portion (310) to thereby maintain the desired structural configuration of body portion (310). Such a cap member may have an appearance similar to that of distal tip member (200).

Distal tip member (300) may also be bent at stem (340) to position the cylindraceous shape defined by rolled body portion (310) coaxially over disk (330). A base member like distal tip base (210) may be interposed between rolled body portion (310) and disk (330), with stem (340) being received in a lateral notch of the base member (e.g., like tab (209) being received in notch (216)). Disk (330) may include electrical circuit components like distal circuit disk (220), with stem (340) providing one or more pathways for electrical communication between the electrical circuit components of disk (330) and electrical circuit components of body portion (310). Body portion (310) may thus be used to apply RF energy to tissue to thereby ablate the tissue. Body portion (310) may also include one or more EP mapping microelectrodes (e.g., like microelectrodes (208)) to thereby provide EP mapping functionality. Openings (320) may be used to expel irrigation fluid from distal tip member (300) after body portion (310) is formed to the desired configuration. Central opening (332) of disk (330) may provide an appropriate path for irrigation fluid to reach the hollow interior of the appropriately formed body portion (310).

Each kind of tip member (200, 300) may also include one or more thermocouples that are configured to provide temperature sensing capabilities. One or more constantan wires may be connected to such thermocouples and may be further connected with corresponding vias in the layers that are proximal to tip member (200, 300) as described herein. In versions where tip member (300) is used, one or more constantan wires may be soldered or otherwise secured to body portion (310) before body portion (310) is bent into a cylindraceous configuration or before peaks (312) are bent inwardly to form a dome configuration. Alternatively, the one or more constantan wires may be secured to respective thermocouples in any other suitable fashion.

In some versions, a distal end of a tether (not shown) may be secured to tip member (200, 300). A proximal end of the tether may be secured to distal tip base (210) or any other component that is proximal to tip member (200, 300). In versions where the proximal end of the tether is secured to a layer that is proximal to distal tip base (210), distal tip base (210) may include a passageway to enable the tether to slidably pass through distal tip base (210). Such a tether may prevent tip member (200, 300) from passing into the patient's bloodstream in the event that tip member (200, 300) becomes separated from distal tip base (210). By way of example only, such a tether may be formed of VECTRAN^{®} by Kuraray Co. of Japan. Various suitable ways in which such a tether may be formed and secured will be apparent to those skilled in the art in view of the teachings herein.

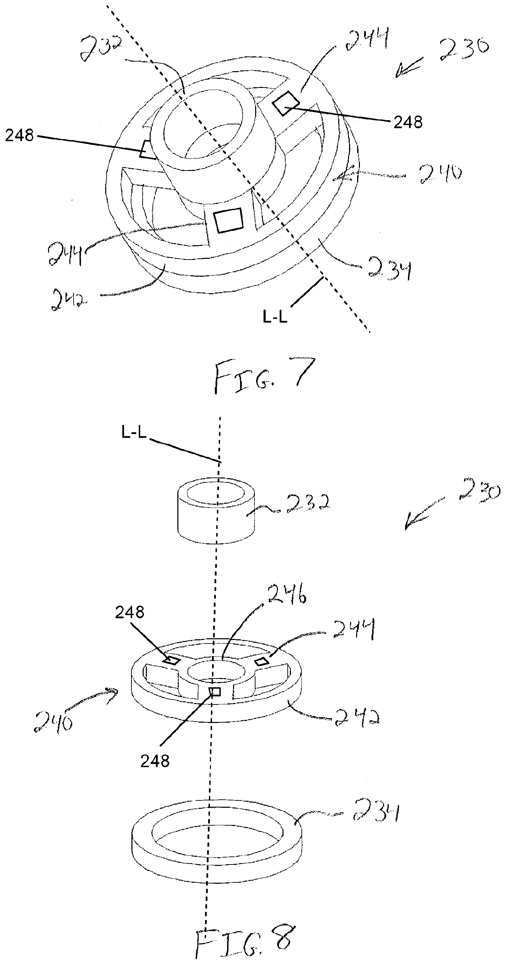

FIGS. 7-8 show strain gauge assembly (230) in greater detail. Strain gauge assembly (230) is positioned proximal to distal circuit disk (220) and is configured to sense external forces that impinge against distal tip member (200). Strain gauge assembly (230) of this example includes a distal column (232), an intermediate body (240), and a proximal ring (234). Intermediate body (240) includes an outer ring (242), an inner ring (246), and three radially extending arms (244) that join rings (242, 246) together. A strain gauge (248) is positioned on each arm (244). Strain gauges (248) may be constructed according to known techniques and may have any suitable configuration to measure axial or bending strains. Strain gauges (248) are configured to sense strain in the corresponding arms (244). From the amount of strain sensed in each of the strain gauges (248), force can be determined from the strain as well as the direction of the force being applied.

Proximal ring (234) has a diameter that corresponds with the diameter of outer ring (242) of intermediate body (240). Proximal ring (234) is fixedly secured to navigation sensor assembly (260) and the layers proximal to navigation sensor assembly (260), such that proximal ring (234) and outer ring (242) cooperate to provide a mechanical ground for strain gauge assembly (230). Distal column (232) has a diameter that corresponds with the diameter of inner ring (246) of intermediate body (240). The distal end of inner ring (246) is fixedly secured to the proximal face of distal circuit disk (220).

When distal tip (200) encounters external forces (e.g., when distal tip (200) is pressed against tissue), those external forces are communicated from distal tip (200) to distal tip base (210), to distal circuit disk (220), and to distal column (232). Distal column (232) further communicates the forces to inner ring (246). When inner ring (246) encounters these forces, while outer ring (242) is mechanically grounded, arms (244) will encounter a tensile strain. This tensile strain will be picked up by strain gauges (248). Thus, tensile strain sensed by strain gauges (248) will be indicative of external forces impinging against distal tip member (200).

The signals from strain gauges (248) may be communicated through vias or other structures in the layers that are proximal to strain gauge assembly (230), eventually reaching first driver module (14) of console (12) via cable (30). First driver module (14) may process the strain signals in accordance with the teachings of various references cited herein; or in any other suitable fashion. By way of example only, console (12) may provide audible feedback to alert the physician (PH) when strain gauges (248) indicate that distal tip member (200) is encountering forces over a predetermined threshold, to thereby prevent the physician (PH) from unwillingly damaging a cardiovascular anatomical structure with distal tip member (200). In some versions, strain gauge assembly (230) may provide consistent force measurements, without compromising effectiveness, when a force up to approximately 100 gram-forces is applied to distal tip member (200).

By way of example only, intermediate body (240) may be constructed of polyether ether ketone (PEEK), polyimide with a rigid stiffener, or liquid crystal polymer with a rigid stiffener. Similarly, distal column (232) or proximal ring (234) may be constructed of polyether ether ketone (PEEK), polyimide with a rigid stiffener, or liquid crystal polymer with a rigid stiffener. By way of further example only, distal column (232) may have a thickness ranging from approximately 0.020 inches to approximately 0.125 inches; an inner diameter of approximately 0.030 inches; and an outer diameter of approximately 0.050 inches. Alternatively, distal column (232) may have any other suitable configuration.

FIGS. 9-10 show navigation sensor assembly (260) in greater detail. FIG. 9 shows navigation sensor assembly (260) in a flat state, before navigation sensor assembly (260) is integrated into end effector (140). FIG. 10 shows navigation sensor assembly (260) in a bent state, which is the state navigation sensor assembly (260) is in when navigation sensor assembly (260) is integrated into end effector (140). Navigation sensor assembly (260) of this example includes a central annular body (262) and a pair of navigation receiver (RX) coil panels (264, 266) coupled to central annular body (262) by respective stems (263, 267). Panels (264, 266) and stems (253, 267) are angularly positioned such that panel (264) and stem (263) are 90 degrees apart from panel (266) and stem (267). When navigation sensor assembly (260) is transitioned from the flat state (FIG. 9) to the bent state (FIG. 10), stems (263, 267) are bent to orient panels (264, 266) at respective angles of 90 degrees from the plane defined by central annular body (262).

Panel (264) includes a RX coil (265), shown schematically in the drawings, that is operable to generate position-indicative electrical signals in response to the alternating magnetic fields generated by field generators (20). Similarly, panel (266) includes a RX coil (269), shown schematically in the drawings, that is operable to generate position-indicative electrical signals in response to the alternating magnetic fields generated by field generators (20). Central annular body (262) can be configured to electrically connect RX coils (265, 269) for transmission (wired or wirelessly) to control system (10). Each RX coil (265, 269) may be formed by electrical traces to define an electrical coil or antenna to receive radiofrequency signals emitted by external transmitters TX coils (e.g., three TX coils provided by field generators (20) positioned external of the patient (PA) body and emitting discrete radiofrequencies) such that the location and orientation of each RX coil (265, 269) can be determined with respect to the TX coils provided by field generators (20).

With navigation sensor assembly (260) in the assembled configuration shown in FIG. 10, the axes of RX coils (265, 269) are orthogonal to each other. Navigation coils (265, 269) may thus together generate signals indicating the position and orientation of end effector (140) in three-dimensional space with substantial precision. The signals from navigation coils (265, 269) may be communicated through vias or other structures in the layers that are proximal to strain navigation sensor assembly (260), eventually reaching first driver module (14) of console (12) via cable (30).

Navigation sensor assembly (260) may be constructed of multiple layers of polyimide or liquid crystal polymer. By way of example only, each layer of navigation sensor assembly (260) may have a thickness ranging from approximately 0.001 inches to approximately 0.004 inches. By way of further example only, the total thickness of navigation sensor assembly (260) may range from approximately 0.004 inches to approximately 0.016 inches. Navigation sensor assembly (260) may be formed as a flex-circuit using any suitable conventional flex-circuit forming techniques.

Central annular body (262) defines a central aperture (268). Central aperture (268) is configured to provide a path for communication of irrigation fluid to the hollow interior of distal tip member (200), as will be described in greater detail below. By way of example only, aperture (268) may have an inner diameter ranging from approximately 0.030 inches to approximately 0.050 inches. In versions where distal circuit disk (220) includes one or more transmission coils to wirelessly communicate electrical signals or power as described above, central annular body (262) may include the complementary coils as described above, such that the coils of central annular body (262) cooperate with the coils of distal circuit disk (220) to provide wireless communication of electrical signals or power across strain gauge assembly (230), without requiring wires, vias, or other electrically conductive structures to pass longitudinally across strain gauge assembly (230). In versions where central annular body (262) includes wireless communication coils, such wireless communication coils may be further coupled with vias or other structures in the layers that are proximal to strain navigation sensor assembly (260), thereby providing a path for electrical communication with first driver module (14) of console (12) via cable (30).

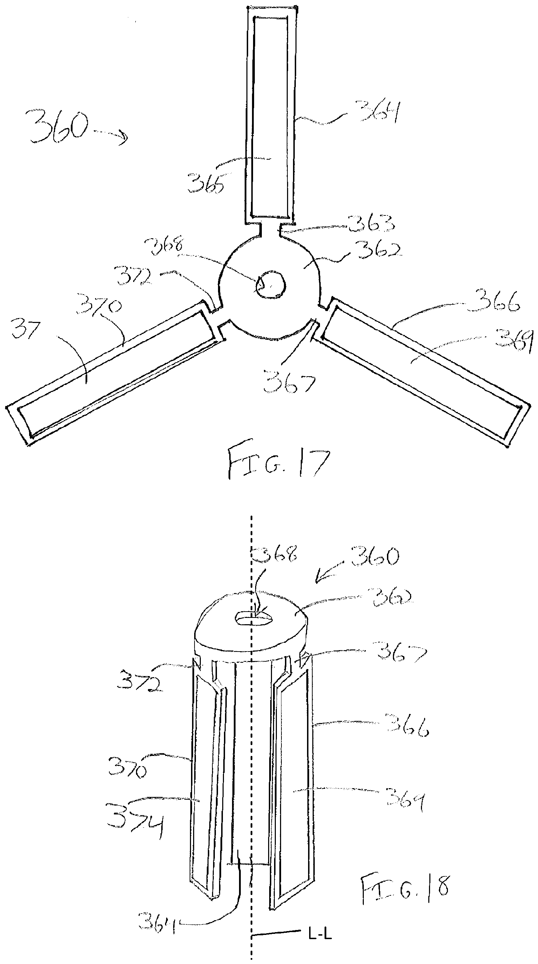

While two receiver coil panels (264, 266) provided in the foregoing example, in some other versions, three receiver coil panels are provided such that each panel is orientated at about 120 degrees with respect to each other as referenced to the central longitudinal axis (L-L). An example of such a version is shown in FIGS. 17-18, which depict an exemplary alternative navigation sensor assembly (360). FIG. 17 shows navigation sensor assembly (360) in a flat state, before navigation sensor assembly (360) is integrated into end effector (140). FIG. 18 shows navigation sensor assembly (360) in a bent state, which is the state navigation sensor assembly (360) is in when navigation sensor assembly (360) is integrated into end effector (140). Navigation sensor assembly (360) of this example includes a central annular body (362) and three navigation receiver (RX) coil panels (364, 366, 370) coupled to central annular body (362) by respective stems (363, 367, 372). Central annular body (362) defines a central aperture (368). Central aperture (368) is configured to provide a path for communication of irrigation fluid to the hollow interior of distal tip member (200).

In the example shown in FIGS. 17-18, panels (364, 366, 370) and stems (363, 367, 372) are angularly positioned such that panels (364, 366, 370) and stems (363, 367, 372) are 120 degrees apart from each other. When navigation sensor assembly (360) is transitioned from the flat state (FIG. 17) to the bent state (FIG. 180), stems (363, 367, 372) are bent to orient panels (364, 366, 370) at respective angles of 90 degrees from the plane defined by central annular body (362). Panel (364) includes a RX coil (365), shown schematically in the drawings, that is operable to generate position-indicative electrical signals in response to the alternating magnetic fields generated by field generators (20). Similarly, panel (366) includes a RX coil (369), shown schematically in the drawings, that is operable to generate position-indicative electrical signals in response to the alternating magnetic fields generated by field generators (20). Panel (370) also includes a RX coil (372), shown schematically in the drawings, that is operable to generate position-indicative electrical signals in response to the alternating magnetic fields generated by field generators (20). Central annular body (362) can be configured to electrically connect RX coils (365, 369, 374) for transmission (wired or wirelessly) to control system (10). Each RX coil (365, 369, 374) may be formed by electrical traces to define an electrical coil or antenna to receive radiofrequency signals emitted by external transmitters TX coils (e.g., three TX coils provided by field generators (20) positioned external of the patient (PA) body and emitting discrete radiofrequencies) such that the location and orientation of each RX coil (365, 369, 374) can be determined with respect to the TX coils provided by field generators (20).

With navigation sensor assembly (360) in the assembled configuration shown in FIG. 18, the axes of RX coils (365, 369, 374) are offset each other. Navigation coils (365, 369, 374) may thus together generate signals indicating the position and orientation of end effector (140) in three-dimensional space with substantial precision. The signals from navigation coils (365, 369, 374) may be communicated through vias or other structures in the layers that are proximal to strain navigation sensor assembly (260), eventually reaching first driver module (14) of console (12) via cable (30).

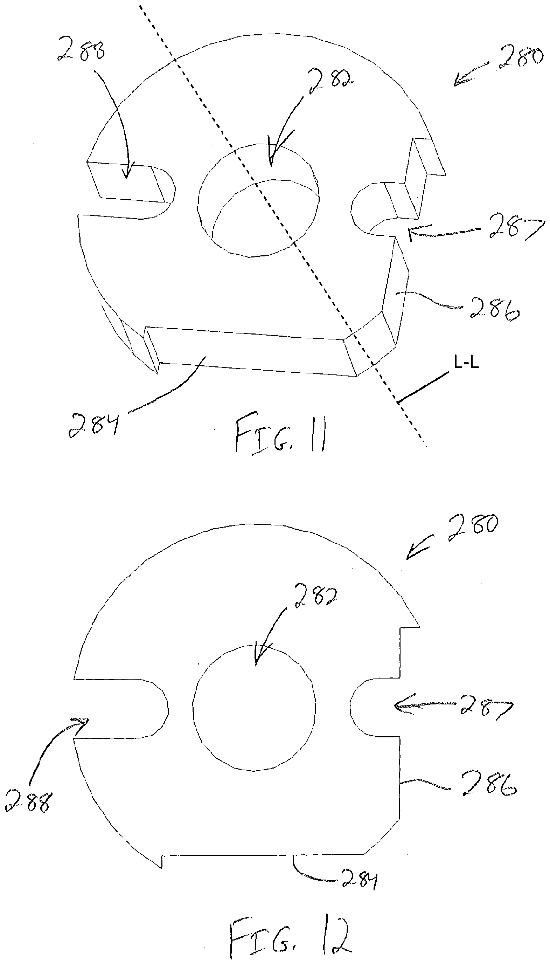

FIGS. 11-14 show distal spacers (280) of distal spacer stack (270) in further detail. In the present example, each distal spacer (280) in distal spacer stack (270) is identical to the rest of the distal spacers (280) in distal spacer stack (270). Each distal spacer (280) is generally shaped like a disk, with a first chordal cutout (284) and a second chordal cutout (286). Each cutout (286) is generally flat. Cutouts (286) are angularly offset from each other by 90 degrees. Cutout (284) is sized and configured to accommodate panel (266) of navigation sensor assembly (260), thereby allowing panel (266) to be radially interposed between distal spacer stack (270) and sheath (122). Cutout (286) is sized and configured to accommodate panel (264) of navigation sensor assembly (260), thereby allowing panel (264) to be radially interposed between distal spacer stack (270) and sheath (122).

Each distal spacer (280) also includes a pair of cable notches (287, 288) extending radially inwardly. Cable notches (287, 288) are angularly offset from each other by 180 degrees. Cable notch (287) is formed in second chordal cutout (286). Each cable notch (287, 288) is configured to receive a respective distal end portion (174, 164) of push-pull cables (170, 172), which will be described in greater detail below. Cable notch (287) and second chordal cutout (286) are configured such that distal end portion (174) of push-pull cable (170) may fit within cable notch (287) while still permitting panel (266) of navigation sensor assembly (260) to fit between distal spacer stack (270) and sheath (122). Each distal spacer (280) further includes a central aperture (282). Central aperture (282) is configured to provide a path for communication of irrigation fluid to the hollow interior of distal tip member (200), as will be described in greater detail below. By way of example only, aperture (282) may have an inner diameter ranging from approximately 0.030 inches to approximately 0.050 inches.

By way of example only, each distal spacer (280) may have a thickness ranging from approximately 0.020 inches to approximately 0.125 inches. By way of further example only, each distal spacer (280) may have an effective outer diameter ranging from approximately 0.055 inches to approximately 0.098 inches. Each distal spacer (280) may be formed of polyether ether ketone (PEEK), polyimide with a rigid stiffener, or liquid crystal polymer with a rigid stiffener. Alternatively, any other suitable material may be used. Each distal spacer (280) may further include one or more vias, traces, or other features that provide one or more pathways for electrical communication between the proximal end of distal spacer stack (270) and the distal end of distal spacer stack (270), as noted above. Distal spacers (280) may be bonded together using any suitable techniques as will be apparent to those skilled in the art in view of the teachings herein.

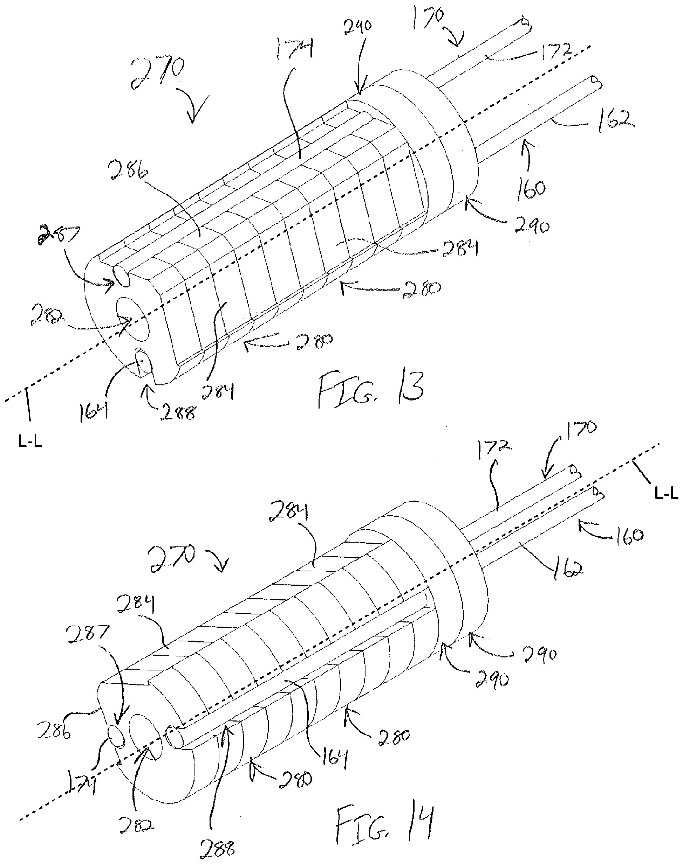

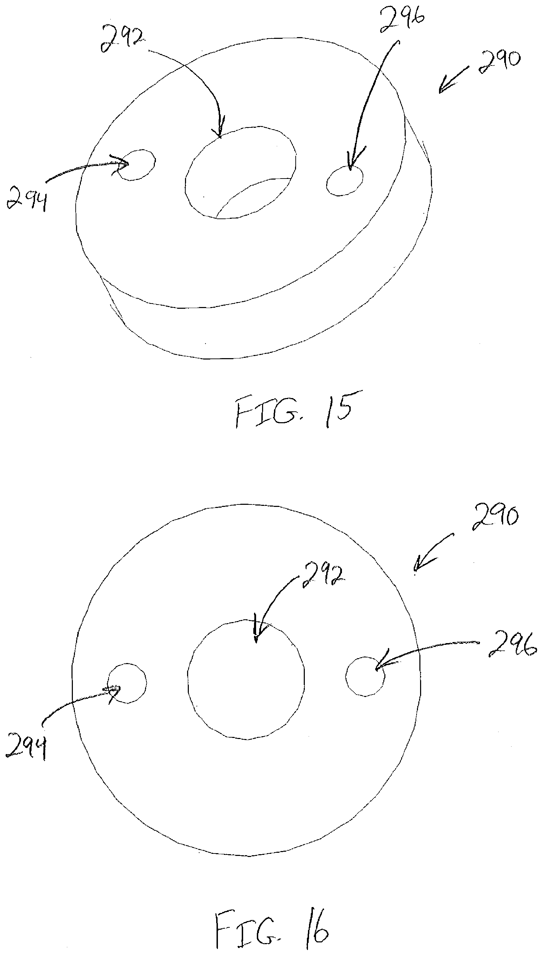

FIGS. 13-16 show proximal spacers (290) in further detail. In the present example, two proximal spacers (290) are provided; proximal spacers (290) are identical to each other. Each proximal spacer (290) is shaped like a disk, with three apertures (292, 294, 296) formed therethrough. Aperture (292) is located at the center of proximal spacer (290) and is configured to provide a path for communication of irrigation fluid to the hollow interior of distal tip member (200), as will be described in greater detail below. By way of example only, aperture (292) may have an inner diameter ranging from approximately 0.030 inches to approximately 0.050 inches. Aperture (294) is sized and configured to receive proximal portion (162) of push-pull cable (160). Aperture (296) is sized and configured to receive proximal portion (172) of push-pull cable (170).

By way of example only, each proximal spacer (290) may have a thickness ranging from approximately 0.020 inches to approximately 0.125 inches. By way of further example only, each proximal spacer (290) may have an effective outer diameter ranging from approximately 0.055 inches to approximately 0.098 inches. Each proximal spacer (290) may be formed of polyether ether ketone (PEEK), polyimide with a rigid stiffener, or liquid crystal polymer with a rigid stiffener. Alternatively, any other suitable material may be used. Each proximal spacer (290) may further include one or more vias, traces, or other features that provide one or more pathways for electrical communication between the proximal end of the proximal side of the proximal-most proximal spacer (290) and the distal end of the distal-most proximal spacer (290), as noted above. Proximal spacers (290) may be bonded together (and to distal spacers (280)) using any suitable techniques as will be apparent to those skilled in the art in view of the teachings herein.

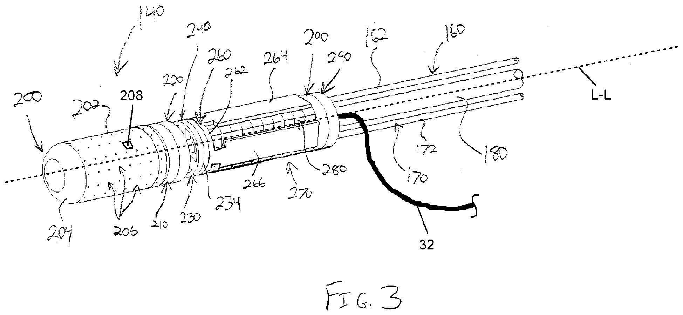

As noted above and as shown in FIGS. 1-2, cable (30) couples catheter assembly (100) with drive system (100). As shown in FIG. 3, wires (32) of cable (30) extend along the length of catheter (120) to reach the proximal-most proximal spacer (290). Wires (32) may thus be contained within sheath (122). Wires (32) may be physically and electrically coupled with the proximal-most proximal spacer (290) in any suitable fashion. By way of example only, the proximal-most proximal spacer (290) may include one or more tabs, with wires (32) being soldered to, or otherwise joined to, such one or more tabs. As another merely illustrative example, the proximal-most proximal spacer (290) may include one or more edge connectors, with wires (32) being soldered to, or otherwise joined to, such one or more edge connectors. As yet another merely illustrative example, a long polyimide tab may extend proximally from the proximal-most proximal spacer (290) and extend along the full length of catheter (120) to a circuit board (or other feature) in handle (110) of catheter assembly (100). Such a long polyimide tab may include traces or other electrically conductive pathways, such that the polyimide tab serves as a flex circuit substituting for wires (32).

As noted above, and as shown in FIGS. 2-4 and 11-14, catheter (100) of the present example further includes a pair of push-pull cables (160, 170). Push-pull cables (160, 170) enable the physician (PH) to selectively deflect end effector (140) laterally away from a longitudinal axis (L-L), thereby enabling the physician (PH) to actively steer end effector (140) within the patient (PA). The proximal ends of push-pull cables (160, 170) may be coupled with a dial, knob, slider, lever, or other user input feature (150) on handle (110). This user input feature (150) may allow the physician (PH) to drive one push-pull cable (160, 170) proximally while the other push-pull cable (160, 170) is simultaneously driven distally. Likewise, the user input feature may allow the physician (PH) to drive one push-pull cable (160, 170) distally while the other push-pull cable (160, 170) is simultaneously driven proximally. The simultaneous, longitudinally-opposing motion of push-pull cables (160, 170) will cause end effector (140) to deflect laterally away from a longitudinal axis (L-L). Various mechanisms that may be used to drive push-pull cables (160, 170) in a simultaneous, longitudinally-opposing fashion will be apparent to those skilled in the art in view of the teachings herein.

Push-pull cable (160) includes a proximal portion (162) and a distal portion (164). Distal portion (164) has a larger outer diameter than the outer diameter of proximal portion (162). In some versions, distal portion (164) is formed by a cylinder that is rigidly secured over the distal end of proximal portion (162). Other suitable ways in which distal portion (164) may be formed will be apparent to those skilled in the art in view of the teachings herein. Push-pull cable (170) includes a proximal portion (172) and a distal portion (174). Distal portion (174) has a larger outer diameter than the outer diameter of proximal portion (172). In some versions, distal portion (174) is formed by a cylinder that is rigidly secured over the distal end of proximal portion (172). Other suitable ways in which distal portion (174) may be formed will be apparent to those skilled in the art in view of the teachings herein.

As noted above, cable notches (287, 288) of distal spacers (280) are configured to receive respective distal end portions (174, 164) of push-pull cables (160, 170). As also noted above, apertures (294, 296) of proximal spacers (290) are configured to receive proximal end portions (162, 172) of push-pull cables (160, 170). Apertures (294, 296) each have an inner diameter that is just large enough to accommodate the outer diameters of proximal end portions (162, 172) of push-pull cables (160, 170); yet the inner diameters of apertures (294, 296) are smaller than the outer diameters of distal end portions (174, 164) of push-pull cables (160, 170). Distal end portions (174, 164) and proximal spacers (290) thus cooperate to prevent push-pull cables (160, 170) from being pulled proximally out of end effector (140). In other words, the proximal face of each distal end portion (174, 164) of push-pull cables (160, 170) will abut the distal face of the distal-most proximal spacer (290). Other suitable ways in which push-pull cables (160, 170) may be coupled with end effector (140) will be apparent to those skilled in the art in view of the teachings herein.

As noted above, catheter assembly (100) is configured to enable irrigation fluid to be communicated from fluid source (42) to catheter (120) via fluid conduit (40), thereby providing expulsion of the irrigation fluid via openings (206) of distal tip member (200). In the present example, the fluid path for the irrigation fluid includes an irrigation tube (180), which is shown in FIGS. 3-4. The proximal end of irrigation tube (180) is coupled with fluid conduit (40) (e.g., at handle (110) of catheter assembly (100)). Irrigation tube (180) extends along the length of catheter (120) to reach end effector (140). By way of example only, irrigation tube (180) may be formed of polyimide or any other suitable flexible material.

In some versions, irrigation tube (180) extends along a substantial portion of the length of end effector (140). For instance, irrigation tube (180) may pass through apertures (292, 282, 268) and the open center of strain gauge assembly (230), ultimately terminating at or in aperture (218) of distal tip base (210). In such versions, irrigation tube (180) may be fixedly attached to distal tip base (210) before distal tip member (200) is formed to have a dome-tipped cylinder shape. As another merely illustrative example, irrigation tube (180) may distally terminate at one of proximal spacers (290). In such versions, irrigation fluid may be communicated from the distal end of irrigation tube (180) through the central passageway formed by the aligned apertures (292, 282, 268), ultimately reaching the interior of distal tip member (200) via aperture (218) of distal tip base (210).

The foregoing description includes various features that may be incorporated into the various layers within end effector (140). By way of further example only, the various layers within end effector (140) may also include op-amps, resistors, capacitors, inductors, LEDs, Bluetooth communication components, WiFi communication components, or various other components. Other components that may be incorporated into one or more of the above-described layers within end effector (140), and various ways in which such components may be incorporated into one or more of the above-described layers within end effector (140), will be apparent to those skilled in the art in view of the teachings herein.

### III. Miscellaneous

It should be understood that any of the examples described herein may include various other features in addition to or in lieu of those described above. By way of example only, any of the examples described herein may also include one or more of the various features disclosed in any of the various references that are acknowledged herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An apparatus, comprising:
(a) a catheter shaft; and
(b) an end effector (140) at a distal end of the catheter shaft, the end effector being sized to fit in an anatomical passageway within a cardiovascular system, the end effector comprising:
(i) a plurality of circuit boards, wherein the circuit boards are stacked longitudinally along a longitudinal axis of the end effector to define a stack of circuit boards, the circuit boards being configured to communicate electrical signals along a full length of the stack of circuit boards, and
(ii) a distal tip member (200), wherein the distal tip member includes at least one electrode electrically coupled to at least one circuit board, the at least one electrode being configured to perform at least one of:
(A) applying RF energy to tissue to thereby ablate the tissue, or
(B) picking up potentials from tissue.

2. The apparatus of claim 1, the distal tip member having a cylindraceous portion (202) and a dome-shaped distal tip (204) at the distal end of the cylindraceous portion, optionally wherein the distal tip member further defines one or more openings (206) configured to expel irrigation fluid.

3. The apparatus of claim 2, wherein the distal tip member further defines one or more openings configured to expel irrigation fluid, and wherein the circuit boards each include at least one passageway configured to enable the irrigation fluid to be communicated through the stack of circuit boards to reach the distal tip member.

4. The apparatus of claim 3, the catheter shaft further comprising an irrigation tube (180) configured to communicate the irrigation fluid along the length of the catheter shaft, optionally wherein the irrigation tube is disposed in one or more of the at least one passageways of the circuit boards.

5. The apparatus of any one or more of claims 1 through 4, the end effector further comprising a force sensor configured to sense forces impinged against the distal tip member, optionally wherein the force sensor comprises a strain gauge assembly (230), the strain gauge assembly being longitudinally interposed between at least some of the circuit boards and the distal tip member.

6. The apparatus of any one or more of claims 1 through 5, the circuit boards being generally disk-shaped.

7. The apparatus of any one or more of claims 1 through 6, the catheter shaft including at least one push-pull cable (160, 170), the at least one push-pull cable being operable to deflect the end effector laterally relative to a longitudinal axis of the catheter shaft, optionally wherein the catheter shaft includes a pair of push-pull cables, a first set of the circuit boards including inwardly extending notches (287, 288) configured to receive respective distal portions of the push-pull cables.

8. The apparatus of any one or more of claims 1 through 7, the end effector further comprising a navigation sensor assembly (260), the navigation sensor assembly being configured to generate signals indicating the position of the end effector in three-dimensional space.

9. The apparatus of claim 8, the navigation sensor assembly comprising:
(A) a central annular body (262),
(B) a first panel (264) extending from the central annular body, the first panel including at least one coil (265), and
(C) a second panel (266) extending from the central annular body, the second panel including at least one coil (269),
and optionally wherein the second panel is angularly offset from the first panel by 90 degrees about the longitudinal axis of the end effector.

10. The apparatus of claim 9, the navigation sensor assembly further comprising a third panel extending from the central annular body, the third panel including at least one coil, and optionally wherein the second panel is angularly offset from the first panel by 90 degrees about the longitudinal axis of the end effector, the third panel being angularly offset from the second panel by 90 degrees about the longitudinal axis of the end effector.

11. The apparatus of any one or more of claims 9 through 10, the catheter shaft including an outer sheath, a first set of the circuit boards including flat regions configured to accommodate the panels between outer regions of the circuit boards of the first set and an inner region of the outer sheath.

12. The apparatus of any one or more of claims 1 through 11, a first circuit board of the plurality of circuit boards including a first communication coil, a second circuit board of the plurality of circuit boards including a second communication coil, the first and second circuit boards being longitudinally spaced apart from each other such that the first and second circuit boards are not in contact with each other, the first and second communication coils being operable to communicate electrical signals wirelessly between the first and second circuit boards.

13. The apparatus of any one or more of claims 1 through 12, the end effector being sized to fit in an anatomical passageway within a human cardiovascular system.

14. The apparatus of claim 1:
wherein the catheter shaft includes an outer sheath; and
wherein the plurality of circuit boards is contained within the outer sheath; and
further comprising a navigation sensor assembly (260) in communication with the circuit boards, the navigation sensor assembly being configured to generate signals indicating a position of the end effector in three-dimensional space; and
wherein the distal tip member is positioned distal to a distal end of the outer sheath, and wherein the distal tip member is in communication with the circuit boards, and wherein the distal tip member is operable to dispense irrigation fluid.

15. The apparatus of claim 14, further comprising a force sensor proximal to the distal tip member, the force sensor being in communication with the circuit boards, the force sensor being configured to sense forces impinging against the distal tip member.

16. A manufacturing method comprising:
(a) arranging a plurality of disk-shaped circuit boards in a longitudinally extending stack, the circuit boards including electrical communication features configured to enable communication of electrical signals from a proximal end of the stack to a distal end of the stack; and
(b) securing a distal tip assembly to the stack, the distal tip assembly including at least one electrode in electrical communication with the electrical communication features of the circuit boards, the at least one electrode being operable to ablate tissue or pick up potentials from tissue.

17. The method of claim 16, further comprising securing at least one push-pull cable (160, 170) to the stack, the at least one push-pull cable being operable to selectively deflect the stack away from a longitudinal axis.

18. The method of any one or more of claims 16 through 17, further comprising securing a navigation sensor assembly (260) to the stack, the navigation sensor being operable to generate signals indicating a position of the distal tip assembly in three-dimensional space.

19. The method of any one or more of claims 16 through 18, further comprising securing a force sensor to the stack, the force sensor being operable to sense forces impinging against the distal tip assembly.

20. The method of any one or more of claims 16 through 19, further comprising a coupling an irrigation tube (180) with the stack, the irrigation tube being operable to communicate irrigation fluid through the stack, the distal tip assembly being operable to expel the irrigation fluid.

## Patentansprüche

1. Vorrichtung, umfassend:
(a) einen Katheterschaft und
(b) einen Endeffektor (140) an einem distalen Ende des Katheterschafts, wobei der Endeffektor so bemessen ist, dass er in einen anatomischen Durchgang innerhalb eines kardiovaskulären Systems passt, wobei der Endeffektor Folgendes umfasst:
(i) eine Vielzahl von Leiterplatten, wobei die Leiterplatten in Längsrichtung entlang einer Längsachse des Endeffektors gestapelt sind, um einen Stapel von Leiterplatten zu definieren, wobei die Leiterplatten so konfiguriert sind, dass sie elektrische Signale über eine volle Länge des Stapels von Leiterplatten kommunizieren, und
(ii) ein distales Spitzenelement (200), wobei das distale Spitzenelement mindestens eine Elektrode umfasst, die elektrisch mit mindestens einer Leiterplatte verbunden ist, wobei die mindestens eine Elektrode so konfiguriert ist, dass sie mindestens eines der Folgenden
durchführt:
(A) Anwenden von HF-Energie auf Gewebe, um dadurch das Gewebe zu ablatieren, oder
(B) Aufnehmen von Potentialen aus Gewebe.

2. Vorrichtung nach Anspruch 1, wobei das distale Spitzenelement einen zylindrischen Abschnitt (202) und eine kuppelförmige distale Spitze (204) am distalen Ende des zylindrischen Abschnitts aufweist, wobei das distale Spitzenelement optional ferner eine oder mehrere Öffnungen (206) aufweist, die zum Ausstoßen von Spülflüssigkeit konfiguriert sind.

3. Vorrichtung nach Anspruch 2, wobei das distale Spitzenelement ferner eine oder mehrere Öffnungen definiert, die zum Ausstoßen von Spülflüssigkeit konfiguriert sind, und wobei die Leiterplatten jeweils mindestens einen Durchgang einschließen, der zum Durchleiten der Spülflüssigkeit durch den Stapel von Leiterplatten zum Erreichen des distalen Spitzenelements konfiguriert ist.

4. Vorrichtung nach Anspruch 3, wobei der Katheterschaft ferner einen Spülschlauch (180) umfasst, der so konfiguriert ist, dass er die Spülflüssigkeit entlang der Länge des Katheterschafts leitet, wobei der Spülschlauch optional in einem oder mehreren der mindestens einen Durchgänge der Leiterplatten angeordnet ist.

5. Vorrichtung nach einem oder mehreren der Ansprüche 1 bis 4, wobei der Endeffektor ferner einen Kraftsensor umfasst, der dazu konfiguriert ist, auf das distale Spitzenelement einwirkende Kräfte zu erfassen, wobei der Kraftsensor optional eine Dehnungsmessstreifenanordnung (230) umfasst, wobei die Dehnungsmessstreifenanordnung in Längsrichtung zwischen mindestens einigen der Leiterplatten und dem distalen Spitzenelement angeordnet ist.

6. Vorrichtung nach einem oder mehreren der Ansprüche 1 bis 5, wobei die Leiterplatten im Allgemeinen scheibenförmig sind.

7. Vorrichtung nach einem oder mehreren der Ansprüche 1 bis 6, wobei der Katheterschaft mindestens ein Druck-/Zugkabel (160, 170) einschließt, wobei das mindestens eine Druck-/Zugkabel betätigbar ist, um den Endeffektor seitlich relativ zu einer Längsachse des Katheterschafts abzulenken, wobei der Katheterschaft optional ein Paar Druck-/Zugkabel einschließt, wobei ein erster Satz der Leiterplatten nach innen verlaufende Kerben (287, 288) einschließt, die dazu ausgelegt sind, jeweilige distale Abschnitte der Druck-/Zugkabel aufzunehmen.

8. Vorrichtung nach einem oder mehreren der Ansprüche 1 bis 7, wobei der Endeffektor ferner eine Navigationssensoranordnung (260) umfasst, wobei die Navigationssensoranordnung dazu konfiguriert ist, Signale zu erzeugen, die die Position des Endeffektors im dreidimensionalen Raum anzeigen.

9. Vorrichtung nach Anspruch 8, wobei die Navigationssensoranordnung Folgendes umfasst:
(A) einen zentralen ringförmigen Körper (262),
(B) eine erste Platte (264), die sich vom zentralen ringförmigen Körper aus erstreckt, wobei die erste Platte mindestens eine Spule (265) einschließt, und
(C) eine zweite Platte (266), die sich vom zentralen ringförmigen Körper aus erstreckt, wobei die zweite Platte mindestens eine Spule (269) einschließt und wobei die zweite Platte optional um 90 Grad um die Längsachse des Endeffektors winkelig von der ersten Platte versetzt ist.

10. Vorrichtung nach Anspruch 9, wobei die Navigationssensoranordnung ferner eine dritte Platte umfasst, die sich von dem zentralen ringförmigen Körper erstreckt, wobei die dritte Platte mindestens eine Spule umfasst und wobei die zweite Platte optional von der ersten Platte um 90 Grad um die Längsachse des Endeffektors winkelig versetzt ist, wobei die dritte Platte von der zweiten Platte um 90 Grad um die Längsachse des Endeffektors winkelig versetzt ist.

11. Vorrichtung nach einem oder mehreren der Ansprüche 9 bis 10, wobei der Katheterschaft eine äußere Hülle einschließt, wobei ein erster Satz der Leiterplatten flache Bereiche einschließt, die so konfiguriert sind, dass sie die Platten zwischen äußeren Bereichen der Leiterplatten des ersten Satzes und einem inneren Bereich der äußeren Hülle aufnehmen.

12. Vorrichtung nach einem oder mehreren der Ansprüche 1 bis 11, wobei eine erste Leiterplatte der Vielzahl von Leiterplatten eine erste Kommunikationsspule einschließt, eine zweite Leiterplatte der Vielzahl von Leiterplatten eine zweite Kommunikationsspule einschließt, wobei die erste und die zweite Leiterplatte in Längsrichtung so voneinander beabstandet sind, dass die erste und die zweite Leiterplatte nicht miteinander in Kontakt stehen, wobei die erste und die zweite Kommunikationsspule betätigbar sind, um elektrische Signale drahtlos zwischen der ersten und der zweiten Leiterplatte zu kommunizieren.

13. Vorrichtung nach einem oder mehreren der Ansprüche 1 bis 12, wobei der Endeffektor so bemessen ist, dass er in einen anatomischen Durchgang im menschlichen kardiovaskulären System passt.

14. Vorrichtung nach Anspruch 1,
wobei der Katheterschaft eine äußere Hülle einschließt und
wobei die Vielzahl von Leiterplatten innerhalb der äußeren Hülle enthalten ist und
ferner umfassend eine Navigationssensoranordnung (260) in Kommunikation mit den Leiterplatten, wobei die Navigationssensoranordnung dazu konfiguriert ist, Signale zu erzeugen, die eine Position des Endeffektors im dreidimensionalen Raum anzeigen; und
wobei das distale Spitzenelement distal zu einem distalen Ende der äußeren Hülle angeordnet ist und wobei das distale Spitzenelement in Kommunikation mit den Leiterplatten steht und wobei das distale Spitzenelement betätigbar ist, um Spülflüssigkeit abzugeben.

15. Vorrichtung nach Anspruch 14, die ferner einen Kraftsensor proximal zum distalen Spitzenelement umfasst, wobei der Kraftsensor mit den Leiterplatten in Kommunikation steht, wobei der Kraftsensor so konfiguriert ist, dass er auf das distale Spitzenelement einwirkende Kräfte erfasst.

16. Herstellungsverfahren, umfassend:
(a) Anordnen einer Vielzahl scheibenförmiger Leiterplatten in einem sich in Längsrichtung erstreckenden Stapel, wobei die Leiterplatten elektrische Kommunikationsmerkmale einschließen, die dazu konfiguriert sind, die Kommunikation elektrischer Signale von einem proximalen Ende des Stapels zu einem distalen Ende des Stapels zu ermöglichen und
(b) Befestigen einer distalen Spitzenanordnung an dem Stapel, wobei die distale Spitzenanordnung mindestens eine Elektrode in elektrischer Kommunikation mit den elektrischen Kommunikationsmerkmalen der Leiterplatten einschließt, wobei die mindestens eine Elektrode betätigbar ist, Gewebe zu ablatieren oder Potenziale von Gewebe aufzunehmen.

17. Verfahren nach Anspruch 16, ferner umfassend das Befestigen von mindestens einem Druck-Zug-Kabel (160, 170) an dem Stapel, wobei das mindestens eine Druck-Zug-Kabel betätigbar ist, den Stapel selektiv von einer Längsachse weg abzulenken.

18. Verfahren nach einem oder mehreren der Ansprüche 16 bis 17, ferner umfassend das Befestigen einer Navigationssensoranordnung (260) an dem Stapel, wobei der Navigationssensor betätigbar ist, um Signale zu erzeugen, die eine Position der distalen Spitzenanordnung im dreidimensionalen Raum anzeigen.

19. Verfahren nach einem oder mehreren der Ansprüche 16 bis 18, ferner umfassend das Befestigen eines Kraftsensors an dem Stapel, wobei der Kraftsensor betätigbar ist, auf die distale Spitzenanordnung einwirkende Kräfte zu erfassen.

20. Verfahren nach einem oder mehreren der Ansprüche 16 bis 19, ferner umfassend ein Koppeln eines Spülschlauchs (180) mit dem Stapel, wobei der Spülschlauch betätigbar ist, Spülflüssigkeit durch den Stapel zu leiten, wobei die distale Spitzenanordnung betätigbar ist, die Spülflüssigkeit auszustoßen.

## Revendications

1. Appareil, comprenant :
(a) une tige de cathéter ; et
(b) un effecteur terminal (140) à l'extrémité distale de la tige du cathéter, l'effecteur terminal étant dimensionné pour s'insérer dans un passage anatomique à l'intérieur d'un système cardiovasculaire, l'effecteur terminal comprenant :
(i) une pluralité de cartes de circuits imprimés, dans lequel les cartes de circuits imprimés sont empilées longitudinalement le long d'un axe longitudinal de l'effecteur terminal pour former une pile de cartes de circuits imprimés, les cartes de circuits imprimés étant configurées pour communiquer des signaux électriques sur toute la longueur de la pile de cartes de circuits imprimés, et
(ii) un élément de pointe distal (200), dans lequel l'élément de pointe distal comporte au moins une électrode couplée électriquement à au moins une carte de circuit imprimé, l'au moins une électrode étant configurée pour effectuer au moins l'une des opérations suivantes
parmi :
(A) l'application de l'énergie RF au tissu pour ablater le tissu, ou
(B) le captage des potentiels des tissus.

2. Appareil selon la revendication 1, l'élément de pointe distal ayant une partie cylindracée (202) et une pointe distale en forme de dôme (204) à l'extrémité distale de la partie cylindracée, éventuellement dans lequel l'élément de pointe distal définit en outre une ou plusieurs ouvertures (206) configurées pour expulser le fluide d'irrigation.

3. Appareil selon la revendication 2, dans lequel l'élément de pointe distal définit en outre une ou plusieurs ouvertures configurées pour expulser le liquide d'irrigation, et dans lequel les cartes de circuit comportent chacune au moins un passage configuré pour permettre au fluide d'irrigation d'être communiqué à travers la pile de cartes de circuit pour atteindre l'élément de pointe distal.

4. Appareil selon la revendication 3, la tige du cathéter comprenant en outre un tube d'irrigation (180) configuré pour communiquer le fluide d'irrigation sur la longueur de la tige du cathéter, éventuellement dans lequel le tube d'irrigation est disposé dans un ou plusieurs de l'au moins un passages des cartes de circuits imprimés.

5. Appareil selon l'une ou plusieurs des revendications 1 à 4, l'effecteur terminal comprenant en outre un capteur de force configuré pour détecter les forces exercées sur l'élément de pointe distale, éventuellement dans lequel le capteur de force comprend un ensemble de jauges de contrainte (230), l'ensemble de jauges de contrainte étant interposé longitudinalement entre au moins certaines des cartes de circuits imprimés et l'élément de pointe distale.

6. Appareil selon l'une ou plusieurs des revendications 1 à 5, les cartes de circuits imprimés ayant généralement la forme d'un disque.

7. Appareil selon l'une ou plusieurs des revendications 1 à 6, la tige du cathéter comportant au moins un câble de poussée et de traction (160, 170), l'au moins un câble de poussée et de traction étant capable de dévier l'effecteur terminal latéralement par rapport à un axe longitudinal de la tige du cathéter, éventuellement dans lequel la tige du cathéter comporte une paire de câbles de poussée et de traction, un premier ensemble de cartes de circuit comportant des encoches s'étendant vers l'intérieur (287, 288) configurées pour recevoir des parties distales respectives des câbles de poussée et de traction.

8. Appareil selon l'une ou plusieurs des revendications 1 à 7, l'effecteur terminal comprenant en outre un ensemble de capteurs de navigation (260), l'ensemble de capteurs de navigation étant configuré pour générer des signaux indiquant la position de l'effecteur terminal dans l'espace tridimensionnel.

9. Appareil selon la revendication 8, l'ensemble du capteur de navigation comprenant :
(A) un corps central annulaire (262),
(B) un premier panneau (264) s'étendant à partir du corps annulaire central, le premier panneau comprenant au moins une bobine (265), et
(C) un deuxième panneau (266) s'étendant à partir du corps annulaire central, le deuxième panneau comportant au moins une bobine (269), et éventuellement dans lequel le deuxième panneau est décalé angulairement de 90 degrés par rapport au premier panneau autour de l'axe longitudinal de l'effecteur terminal.

10. Appareil selon la revendication 9, l'ensemble de capteurs de navigation comprenant en outre un troisième panneau s'étendant à partir du corps annulaire central, le troisième panneau comprenant au moins une bobine, et éventuellement dans lequel le deuxième panneau est décalé angulairement du premier panneau de 90 degrés par rapport à l'axe longitudinal de l'effecteur terminal, le troisième panneau étant décalé angulairement du deuxième panneau de 90 degrés par rapport à l'axe longitudinal de l'effecteur terminal.

11. Appareil selon l'une ou plusieurs des revendications 9 à 10, la tige du cathéter comportant une gaine extérieure, un premier ensemble de cartes de circuits imprimés comportant des régions plates configurées pour accueillir les panneaux entre les régions extérieures des cartes de circuits imprimés du premier ensemble et une région intérieure de la gaine extérieure.

12. Appareil selon l'une ou plusieurs des revendications 1 à 11, une première carte de circuit de la pluralité de cartes de circuit comportant une première bobine de communication, une deuxième carte de circuit de la pluralité de cartes de circuit comportant une deuxième bobine de communication, la première et la deuxième carte de circuit étant espacées longitudinalement l'une de l'autre de sorte que la première et la deuxième carte de circuit ne sont pas en contact l'une avec l'autre, la première et la deuxième bobine de communication étant aptes à communiquer des signaux électriques sans fil entre la première et la deuxième carte de circuit.

13. Appareil selon l'une ou plusieurs des revendications 1 à 12, l'effecteur terminal étant dimensionné pour s'insérer dans un passage anatomique du système cardiovasculaire humain.

14. Appareil selon la revendication 1,
dans lequel la tige du cathéter comporte une gaine extérieure ; et
dans lequel la pluralité de circuits imprimés est contenue dans la gaine extérieure ;
et
comprenant en outre un ensemble de capteurs de navigation (260) en communication avec les cartes de circuits imprimés, l'ensemble de capteurs de navigation étant configuré pour générer des signaux indiquant une position de l'effecteur terminal dans l'espace tridimensionnel ; et
dans lequel l'élément de pointe distal est positionné distalement par rapport à l'extrémité distale de la gaine
Extérieure, et dans lequel l'élément de pointe distal est en communication avec les
cartes de circuits imprimés, et dans lequel l'élément de pointe distal peut être utilisé pour distribuer un fluide d'irrigation.

15. Appareil selon la revendication 14, comprenant en outre un capteur de force à proximité de l'élément de pointe distal, le capteur de force étant en communication avec les cartes de circuit, le capteur de force étant configuré pour détecter les forces s'exerçant sur l'élément de pointe distal.

16. Procédé de fabrication comprenant :
(a) l'agencement d'une pluralité de cartes de circuits imprimés en forme de disque dans une pile s'étendant longitudinalement, les cartes de circuits imprimés comportant des caractéristiques de communication électrique configurées pour permettre la communication de signaux électriques d'une extrémité proximale de la pile à une extrémité distale de la pile ; et
(b) la fixation d'un ensemble de pointe distale à la pile, l'ensemble de pointe distale comportant au moins une électrode en communication électrique avec les caractéristiques de communication électrique des cartes de circuits imprimés, l'au moins une électrode pouvant être utilisée pour ablater un tissu ou capter des potentiels à partir d'un tissu.

17. Procédé selon la revendication 16, comprenant en outre la fixation d'au moins un câble de poussée et de traction (160, 170) à la pile, l'au moins un câble de poussée et de traction pouvant être utilisé pour dévier sélectivement la pile d'un axe longitudinal.

18. Procédé selon l'une ou plusieurs des revendications 16 à 17, comprenant en outre la fixation d'un ensemble de capteurs de navigation (260) à la pile, le capteur de navigation pouvant être utilisé pour générer des signaux indiquant une position de l'ensemble de pointe distale dans l'espace tridimensionnel.

19. Procédé selon l'une ou plusieurs des revendications 16 à 18, comprenant en outre la fixation d'un capteur de force à la pile, le capteur de force pouvant être utilisé pour détecter les forces s'exerçant sur l'ensemble de pointe distale.

20. Procédé selon l'une ou plusieurs des revendications 16 à 19, comprenant en outre un couplage de tube d'irrigation (180) avec la pile, le tube d'irrigation pouvant être utilisé pour communiquer un liquide d'irrigation à travers la pile, l'ensemble de pointe distale pouvant être utilisé pour expulser le fluide d'irrigation.
